# EUROPEAN PATENT APPLICATION

(11) **EP 2 801 339 A1**
(43) Date of publication of application: **12.11.2014**
(21) Application number: 13382250.2
(22) Date of filing: 27.06.2013
(51) Int. Cl.: A61F 2/32, A61F 2/34, A61F 2/36, A61F 2/46, A61F 2/30

(54) **Improved hip replacement**

(30) Priority: 09.05.2013 ES 201330553 U
(71) Applicant: Florian Argüez, Jose, 11300 La Linea de la Concepcion, Cadiz (ES)
(72) Inventor: Florian Argüez, Jose, 11300 La Linea de la Concepcion, Cadiz (ES)
(74) Representative: Bartrina Diaz, José María

(57) **Abstract**

Improved hip replacement consists in the insertion of a replacement rod into the femoral condyle. This gives the pelvic bone access to the acetabular cup supporting the condyle through an improved hip replacement implant based on its rotation using ball joints and a bone attachment system; and using a screw spike inserted in the femur and fixing it to the rectangular face bushing on the outer surface of said bone to which the condyle is also rigidly joined. The condyle is inserted in a round configuration consisting of the round shell in contact with the pelvic bone and the threaded sealing lid, the place where the ball joints that guarantee rotation between said bones are located. Finally, said round shell in contact with the pelvic bone is completed with filaments for securing it to said bone.

## Description

### PURPOSE OF THE INVENTION

This invention refers to complete hip joint replacement entailing the insertion of a replacement rod into the femoral condyle. This gives the pelvic bone access to the acetabular cup supporting the condyle through an improved hip replacement implant based on its rotation using ball joints and a bone attachment system. This also applies to shoulder joints or other parts of the body where constant rotation is required.

### BACKGROUND OF TECHNOLOGY

The hip joint is one of the largest in the body and it is used to support its weight. It consists of a cup (acetabulum) and ball (head of the femur) enclosed in a capsule that seals and stabilises the joint. This helps to keep the hip stable during rotation and other movements, even in extreme ranges of motion. A healthy hip allows you to walk, kneel down and turn around without feeling pain.

The hip joint is formed where the round end of the muscle bone (head of the femur) joins the pelvic bone (acetabulum). The joint is covered with tissues and moved by powerful muscles. When all these parts are healthy, the hip should move easily.
- Cartilage is a smooth coat of tissue covering the head of the muscle bone (femur) and also the pelvic bone cup (acetabulum). Healthy cartilage absorbs stress and enables the head of the femur to move easily in the pelvic cup.
- Muscles move the hip and legs.
- Tendons fasten muscles to the bones.

The hip is one of the joints that supports most body weight. As it is a round joint, the hip can remain stable when the body bends over or rotates or during extreme ranges of motion. A healthy hip allows you to walk, kneel down and turn around without feeling pain but if you hurt yourself, it is most likely to be painful when you move. A prosthesis is used to replace a natural hip.

In a healthy hip, the ends of the femur and pelvis (meeting point between the hip and the femur) are covered with smooth cartilage. This enables the ball to slide easily inside the cup. When nearby muscles support the weight and joints move freely, you can walk without feeling pain.

On the other hand, with a damaged hip, the cartilage wears away and no longer acts as a buffer. Due to constant friction, brittle bones become irregular in shape and their surface looks like sand paper. The head of the femur scrapes against the acetabulum when you move your leg, causing pain and stiffness.

Due to the status of techniques used in this area, a prosthesis consists of a round artificial ball that replaces the head of the femur and an artificial cup replaces the acetabulum. A rod is inserted inside the bone to make it stable. These parts are joined together to create your new artificial joint. The parts have smooth surfaces in areas that join together to facilitate movement and reduce wear and tear. In areas where they join the bone, there might be a rough surface for the bone to grip the prosthesis.

The head of the femur (muscle bone) is cut and the pelvic cup (cup or acetabulum) is prepared with semicircular canals, leaving a cavity for the prosthesis cup (cotyloid cup). Generally, the cotyloid cup is pressed into the pelvis but it is sometimes secured in place with screws or cement. Press-in prostheses contain tiny pores on the surface, allowing the bone to grow inside. They are known as prostheses with a porous coating or non-cemented, in comparison with cemented prostheses that are fixed to the bone with bone cement.

The new hip joint rod is inserted into the upper part of the femur. Once the rod is well-secured, it is joined to the "ball or prosthesis head" that, in turn, joins the cotyloid cup on the prosthesis (the new cup or cavity). The prosthesis rod can also be secured with cement or can even be pressed in and covered with a pore where the bone will grow.

However, the techniques described have the following disadvantages:
- When emptying the bone and then filling it up with cement, it becomes weak. If the patient then suffers from necrosis in the area, the only solution is to cut away the whole affected part, which reduces the possibility of receiving a new prosthesis. In fact, if this occurs more than twice, it is impossible to insert a new implant.
- The prosthesis ball is inserted in direct contact with the receiving bone causing extreme friction and therefore aches and pains and the corresponding wear and tear of said bone.
- Sometimes, the implant may come out from hip itself, causing very painful dislocation that is harmful to the patient.

Based on the problems mentioned above, the "Improved hip replacement" has the following advantages considering the status of the technique:
- The prosthesis rod is inserted into the bone to a "corkscrew" type screw to avoid previous emptying of the bone and the consequent inconveniences mentioned above.
- Therefore, when it is screwed into the bone, in case of necrosis or any other problems, it can be removed without having to damage the bone or cut it.
- On the upper part of the prosthesis rod, before it is joined to the ball, there is a rectangular structure or support cap that varies in length according to the size of the bone, which is hollow underneath, allowing for the bone to be inserted but gripping it and securing it on the outside to make it more stable, preventing it from breaking in this area and allowing the patient to move more comfortably.
- The upper round or condyle part consists of a ball that moves inside a round cap using ball joints to avoid wear and tear through bone friction mentioned above. Theouter part of the round cap has filaments, which are inserted into the hip bone and secured to it to avoid friction and dislocation.
- Joint balls movement inside the cap can be adjusted using components in the area that guarantee the appropriate adjustment.

### EXPLANATION OF THE INVENTION

The "Improved hip replacement" proposed by the invention completely solves previous hip problems and entails securing it to the femur using a screw spike inserted in the femur and fixing it to the rectangular face bushing on the outer surface of said bone to which the condyle is also rigidly joined.

The condyle is inserted in a round configuration consisting of the round shell in contact with the pelvic bone and the threaded sealing lid, the place where the ball joints that guarantee rotation between said bones are located.

Finally, said round shell in contact with the pelvic bone is completed with filaments for securing it to said bone.

### DESCRIPTION OF THE DRAWINGS

To complete this description and for a better understanding of the characteristics of the invention, in terms of a preferred practical example, a set of drawings is included as an integral part of it, showing the following:
Figure 1.- General view of the "Improved hip replacement" units after the operation.
Figure 2.- View of the main section of the "Improved hip replacement" set.
Figure 3.- Main view of the disassembly of the disassembly of the screw bushing and condyle unit.
Figure 4.- Main view of the disassembly of the screw bushing and condyle unit using a torque wrench.
Figure 5.- Useful as a hammer according to the interlocking position of the unit on the pelvic bone.
Figure 6.- General view of the "Improved hipreplacement" unit that includes replacement of the end of the femur if it is too damaged for use.

In the figures above, it is important to mention the following elements:
1. Femur.
2. Pelvic bone.
3. Acetabular cup.
4. Screw inserted into the femur.
5. Final face bushing of the screw on the femur.
6. Condyle.
7. Round shell in contact with the pelvic bone.
8. Ball joint location lid.
9. O-ring seal.
10. Ball joints.
11. Filaments for securing the pelvic bone.
12. Reinforcement of joint between screw and condyle.
13. Holes for use of torque wrench.
14. Useful as a hammer for interlocking the unit.
15. Titanium part to replace head of femur.

### EXAMPLE OF PREFERRED EMBODIMENT

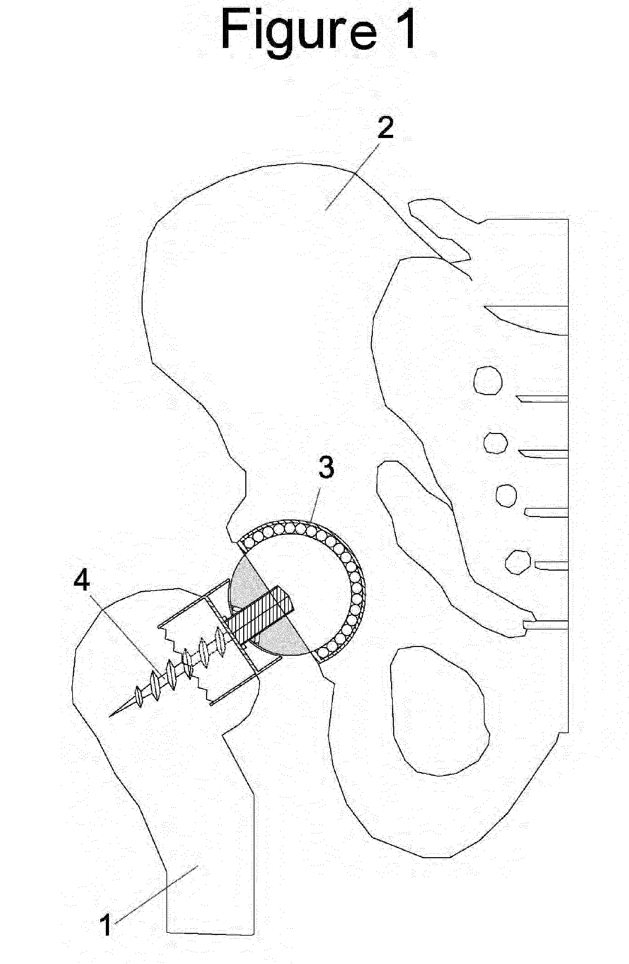

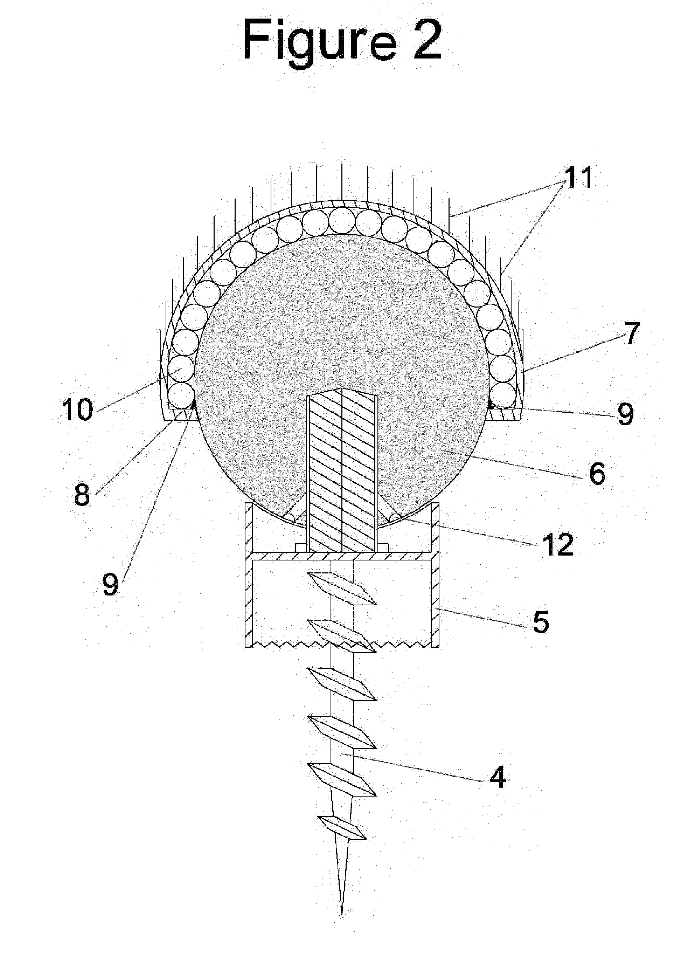

Preference is given to the manufacture of the "Improved hip replacement" in titanium to be used to replace the head of the femur (1) in its articulation with the pelvic bone (2) as shown in Figures1 and 2.

This is how the prosthesis invented is housed in the acetabular cup (3) and comprises the screw spike for securing it to the femur (4) that is inserted into the femur (1) and the face bushing (5) on the outer surface of said bone rigidly joined to the condyle (6).

The condyle (6) is inserted into a round configuration consisting of the round shell (7) in contact with the pelvic bone and the threaded sealing lid (8) equipped with its corresponding o-ring seal (9), where the stainless steel ball joints (10) that guarantee rotation between said bones are located.

Finally, said round shell (7) in contact with the pelvic bone (2) is completed with filaments (11) measuring two millimetres in diameter and long enough to guarantee fixture to said bone.

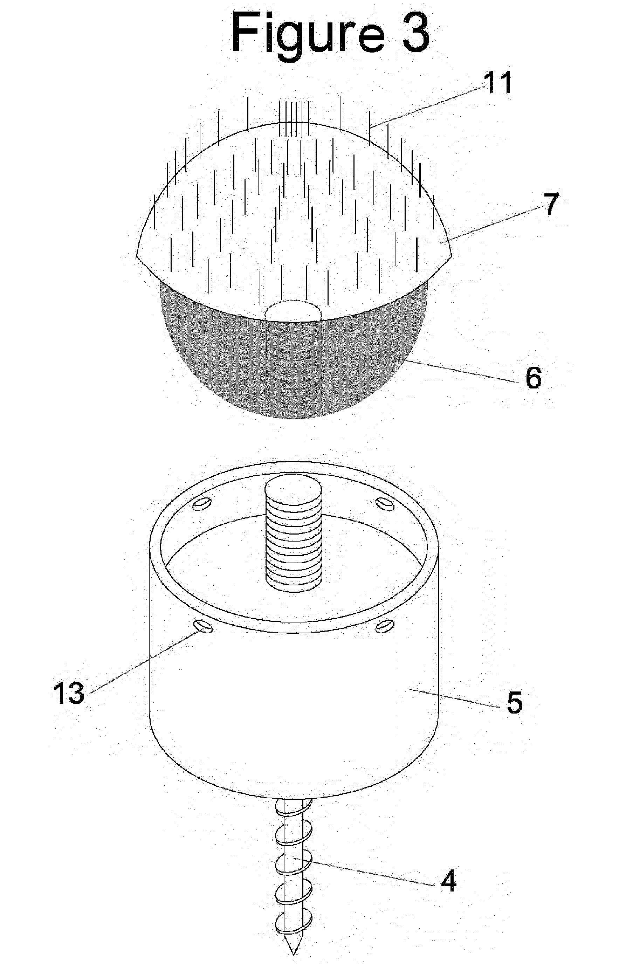

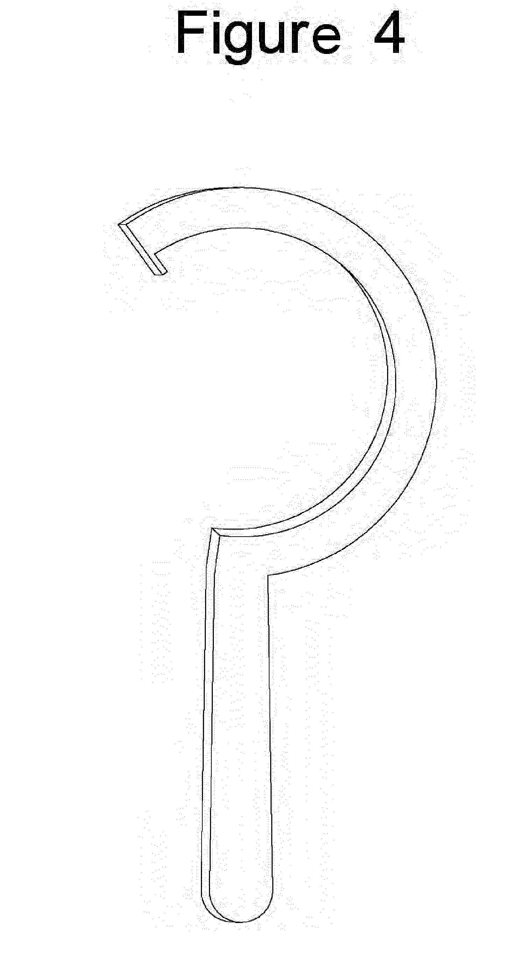

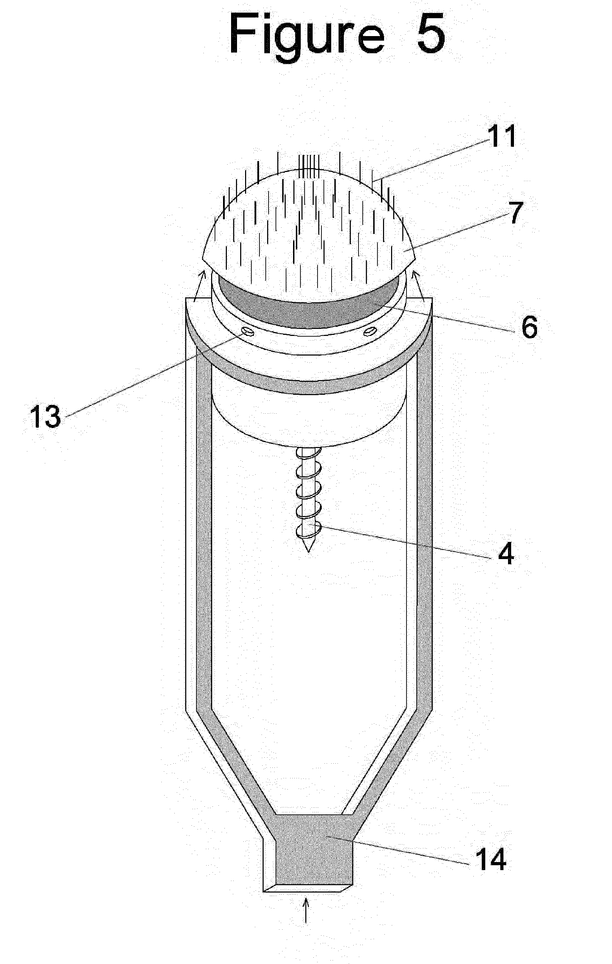

Finally, figures 3, 4 and 5 show the corresponding wrenches or accessories, used to join together or secure the different elements involved.

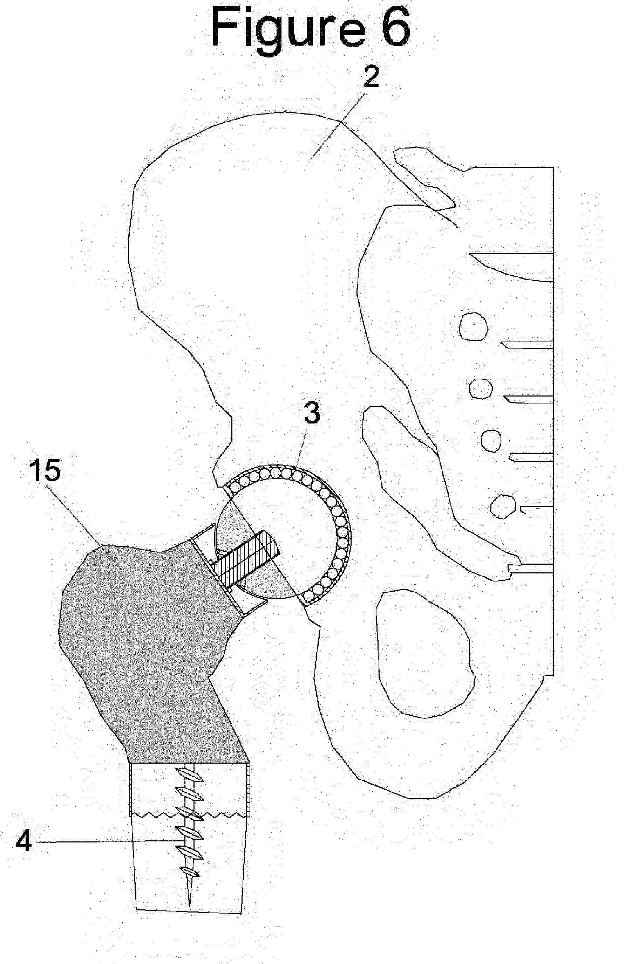

In the alternative method, shown in figure 6, there is sometimes bone deterioration in the access area to the femur that requires the screw (4) to be formed with two axes using an intermediate part to replace the head of the femur (15) to guarantee access to the bone in a healthy area or supply sufficient resistance.

It is not considered necessary to expand on this description for any expert on the subject to understand the scope of the invention and its advantages. The materials used, dimensions, technical solutions proposed to guarantee fixture of the bushing (5), adjustment of the lid (8) or its application, shall be subject to alteration whenever this does not imply altering the basic principles of the invention.

## Claims

1. "Improved hip replacement" **characterised by** elements used to secure it to the femur such as a screw spike and a bushing for ecuring it to the outer surface of said bone, to which the condyle is rigidly joined.

2. "Improved hip replacement" according to claim 1, **characterised by** elements for use in articulation such as ball joints according to a condyle in a round configuration, an outer coating or round shell in contact with the pelvic bone and the threaded lid, where the ball joints that guarantee rotation between said bones are located.

3. "Improved hip replacement" according to claim 1 and 2, **characterised by** its round shell or coating in contact with the pelvic bone completed with filaments used for securing said bone.

4. "Improved hip replacement" according to claim 1, 2 and 3, characterised because the unit may otherwise be formed by two main axes with an intermediate part replacing the head of the femur (15).
